# EUROPEAN PATENT APPLICATION

(11) **EP 2 997 969 A1**
(43) Date of publication of application: **23.03.2016**
(21) Application number: 14798079.1
(22) Date of filing: 01.05.2014
(51) Int. Cl.: A61K 33/08, A61K 31/716, A61P 1/10

(54) **TREATMENT AGENT AND TREATMENT METHOD FOR INTESTINAL EXAMINATION OR SURGERY**

(30) Priority: 17.05.2013 JP 2013104988
(71) Applicant: Kyowa Chemical Industry Co., Ltd, Takamatsu-shi, Kagawa 761-0113 (JP)
(72) Inventor: YOSHIMURA, Yuya, Kita-gun Kagawa 761-0705 (JP); ISHIHAMA, Satoru, Kita-gun Kagawa 761-0705 (JP)
(74) Representative: Raynor, Stuart Andrew
(86) International application number: PCT/JP2014/062088
(87) International publication number: WO 2014/185283

(57) **Abstract**

A treating agent for the examination or operation of the large intestine which has high examination precision or facilitates an operation and can be administered with a small amount of water. The treating agent for the examination or operation of the large intestine contains magnesium oxide particles and is used to be orally administered before an examination or operation.

## Description

### TECHNICAL FIELD

The present invention relates to a treating agent for the examination or operation of the large intestine. More specifically, it relates to a treating agent which ejects the contents of the large intestine completely to clean it, improves examination precision and enables a safe operation when it is ingested before the examination or operation of the large intestine.

### BACKGROUND ART

In recent years, due to an increase in the number of patients who suffer from a polyp or tumor in the large intestine, the number of examinations or operations for them has been growing accordingly. The examination of the large intestine is carried out by CT examination, endoscopy or X-ray examination in which the complete ejection of contents in the intestinal tract is essential to the diagnosis of a disease. Therefore, a subject has to be on dietary restrictions and take a treating agent for the cleaning of the intestinal tract before the examination.

As the treating agent for the cleaning of the intestinal tract, a PEG solution containing polyethylene glycol and an electrolyte as main components has been used. As a substitute for the PEG solution, an aqueous solution containing magnesium citrate, an electrolyte and a saccharide (to be referred to as "magnesium citrate solution" hereinafter) has been developed. This magnesium citrate solution is proposed in the following Patent Document 1.

Although the PEG solution and the magnesium citrate solution have a high intestinal tract cleaning effect, to achieve this effect, a large amount (about 2 liters or more) of the solution must be ingested within a time limit. Even when the solution is administered in several times, a subject is forced to have a burden or pain. Although the intestinal tract cleaning effect of the treating agent is high, when the treating agent is ingested, it significantly increases the frequency of defecation, thereby making it difficult for the subject to have enough rest before an examination or operation. Further, the treating agent has a disadvantage that it deteriorates examination precision due to remaining water in the intestinal tract.

### Prior Art Documents

Patent Document 1: JP-A 2003-183155

### DISCLOSURE OF THE INVENTION

### Problem to be Solved by the Invention

The inventors of the present invention carried out studies on the development of a treating agent for the examination or operation of the large intestine, which has a high intestinal tract cleaning effect and can improve receptivity when a relatively small amount of a liquid is administered.

As a result, they found that when a treating agent containing magnesium oxide particles as the main component is orally administered, the amount of water to be taken can be greatly reduced, contents in the intestinal tract can be ejected almost completely, and the particles can be administered in the form of a tablet or powder, thereby making it possible to greatly improve the receptivity of a subject. As a result of ingestion, it was observed that the amount of remaining water in the intestinal tract can be greatly reduced. It was also found that a small amount of the remaining water is effective especially in CT examination.

The inventors of the present invention also found from further research that when a treating agent containing magnesium oxide particles and water-soluble dietary fibers as effective components is ingested, the intestinal tract cleaning effect becomes high and receptivity can be improved with a relatively small amount of a liquid. As a result, they developed a treating agent for the examination or operation of the large intestine, which has a high intestinal tract cleaning effect by the administration of a relatively small amount of a liquid, can greatly reduce the amount of water to be taken, can eject contents in the intestinal tract almost completely without greatly increasing the frequency of defecation and can improve receptivity when the magnesium oxide particles and the water-soluble dietary fibers are orally administered. It was revealed that this treating agent can greatly reduce the amount of water to be taken, can eject contents in the intestinal tract almost completely and can eject contents in the intestinal tract without greatly increasing the frequency of defecation when magnesium oxide particles are used in combination with water-soluble dietary fibers and that this combination can improve the receptivity of a subject as it can be administered in the form of a granule, tablet or oral liquid agent. It was found that this combination is free from interaction and greatly improves a defecation effect as well as an intestinal tract cleaning effect due to a synergistic effect. It was also found that these effects are very high especially in CT examination.

### Means for Solving the Problem

The present invention has been accomplished based on the above finding, and according to the present invention, there are provided a treating agent (A) and a treating agent (B) for the examination or operation of the large intestine described below.

### Treating agent (A);

A-(1) treating agent (A) for the examination or operation of the large intestine, which contains 88 to 97 wt% of magnesium oxide particles and is used to be orally administered before an examination or operation.
A- (2) treating agent (A) according to A-(1) above, wherein the examination of the large intestine is CT examination, endoscopy or X-ray examination.
A-(3) treating agent (A) according to A-(1) above, which contains 1.5 to 6 g of the magnesium oxide particles for each examination or each operation.
A-(4) treating agent (A) according to A-(1) above, which contains the magnesium oxide particles having a one-time acid-neutralizing capacity of 150 ml or more as the consumption of 0.1 N hydrochloric acid.
A-(5) treating agent (A) according to A-(1) above, wherein the magnesium oxide particles have an average secondary particle diameter of 0.5 to 10 µm.
A-(6) treating agent (A) according to A-(1) above, which is a tablet, capsule, granule, powder, oral liquid, syrup or oral jelly containing the magnesium oxide particles.
A-(7) treating agent (A) according to A-(1) above, which is a tablet containing magnesium oxide particles as the main component and having (i) an average secondary particle diameter of the magnesium oxide particles contained therein of 0.5 to 10 µm, (ii) a content of the magnesium oxide particles contained therein of 88 to 97 wt% and (iii) a disintegration time of 10 seconds or less.

### treating agent (B);

B-(1) treating agent (B) for the examination or operation of the large intestine, which contains magnesium oxide particles and water-soluble dietary fibers as effective components and is used to be orally administered before an examination or operation.
B-(2) treating agent (B) according to B-(1) above, wherein the examination of the large intestine is CT examination, endoscopy or X-ray examination.
B-(3) treating agent (B) according to B-(1) above, which contains 1.5 to 6 g of the magnesium oxide particles and 5 to 20 g of the water-soluble dietary fibers for each examination or each operation.
B-(4) treating agent (B) according to B-(1) above, which contains the magnesium oxide having a one-time acid-neutralizing capacity of 150 ml or more as the consumption of 0.1 N hydrochloric acid.
B-(5) treating agent (B) according to B-(1) above, wherein the magnesium oxide particles have an average secondary particle diameter of 0.5 to 10 µm.
B-(6) treating agent (B) according to B-(1) above, wherein the water-soluble dietary fibers contained in the treating agent (B) are at least one selected from the group consisting of reduced indigestible dextrin, indigestible dextrin and polydextrose.
B-(7) treating agent (B) according to B-(1) above, which is a tablet, capsule, granule, powder, oral liquid, syrup or oral jelly containing the magnesium oxide particles and the water-soluble dietary fibers as effective components.

### treating method;

According to the present invention, there are further provided the following treating methods [(C)-1 to (C)-4] for carrying out the examination or operation of the large intestine using the above treating agent (A) and the treating agent (B). (C)-1: treating method for carrying out the examination or operation of the large intestine after the above treating agent (A) or the above treating agent (B) is orally administered.
(C)-2: treating method according to (C)-1 above, wherein the examination of the large intestine is CT examination, endoscopy or X-ray examination.
(C)-3: treating method according to (C)-1 above, wherein the above treating agent (A) or the above treating agent (B) containing 1.5 to 6 g of the magnesium oxide particles for each examination or each operation is orally administered.
(C)-4: treating method according to (C)-1 above, wherein the above treating agent (A) or the above treating agent (B) is orally administered together with 600 to 1,500 ml of water for each examination or each operation.

### Effect of the Invention

Since all of the treating agent (A) and the treating agent (B) for the examination or operation of the large intestine according to the present invention contain magnesium oxide particles, when it is orally administered before an examination or operation, it can eject contents and water in the intestinal tract almost completely, thereby making it possible to increase various examination precisions with a relatively small amount of a liquid (water), and does not cause any troubles during an operation. Since the magnesium oxide particles have high receptivity and can be taken with a small amount of a liquid, they have an advantage that a burden or pain on a subject is little.

### BEST MODE FOR CARRYING OUT THE INVENTION

The examination of the large intestine is used to check the growth or existence of a polyp or tumor on the inner wall of the large intestine and generally carried out by CT examination, endoscopy or X-ray examination. The precisions of these examinations are greatly influenced by the existence or amounts of remaining feces or remaining water in the intestinal tract. The remaining feces or remaining water should be nonexistent during an operation.

Therefore, conventional intestinal tract cleaning agents for the examination or operation of the large intestine require the ingestion of a large amount, i.e., 2 liters or more of water within a time limit, which inflicts a great pain on a subject.

The treating agent (A) of the present invention contains magnesium oxide particles as the main component. As for the treating agent of the present invention, it is desirable that 1. 5 to 6 g, preferably 2 to 5 g of the magnesium oxide particles should be orally administered to each person for each examination or each operation. At this point, the amount of water is about 600 to 1, 500 ml, preferably 900 to 1,200 ml. The above-specified amounts of the magnesium oxide particles and water are the total amounts used for each treatment (examination or operation) and may be generally divided into two or three portions to be administered. For example, when they are divided into three portions to be administered, 0.5 to 2 g of the magnesium oxide particles should be administered together with 200 to 500 ml of water each time. When they are divided into portions to be administered, the portions of the magnesium oxide particles and water to be administered each time do not need to be the same in quantity and may be different.

Since the treating agent (A) of the present invention contains the magnesium oxide particles as the main component, even when the amount of water to be orally taken is relatively small, it has excellent defecation and water ejection effects. For example, even when the total amount of water is about 1,500 ml or less for each treatment, the purpose can be completely accomplished.

Since the treating agent (A) of the present invention contains the magnesium oxide particles as the main component, it exhibits antacid action in the stomach to neutralize gastric acid, and magnesium chloride produced by neutralization becomes a bicarbonate inside the intestine to exhibit laxative action. Therefore, as a treating agent having minimum antacid action produced by neutralization, the agent desirably contains magnesium oxide particles having an acid-neutralizing capacity for each treatment of 150 ml or more, preferably 700 to 3,000 ml as the consumption of 0.1 N hydrochloric acid.

The magnesium oxide particles used as the treating agent have an average secondary particle diameter measured by a laser diffraction scattering method of 0.5 to 10 µm, preferably 1 to 7 µm.

Although the treating agent (A) of the present invention may contain magnesium oxide particles in various forms as the main component, it is desirably any one of a tablet, capsule, granule, powder, oral liquid, syrup and oral jelly. Out of these, it is preferably a tablet from the viewpoints of ease of taking, formulation ease and preservability.

The tablet containing the magnesium oxide particles is advantageous when (i) the average secondary particle diameter of the magnesium oxide particles contained therein is 0.5 to 10 µm and (ii) the content of the magnesium oxide particles contained therein is 88 to 97 wt%, preferably 89 to 96 wt%. Further, when the tablet has an excellent disintegration property, it is easy to take and the amount of water to be taken is small advantageously. The disintegration time is preferably 10 seconds or less.

Therefore, for tabletting, it is desirable to use sodium carboxymethyl cellulose, less-substituted hydroxypropyl cellulose or crystalline cellulose as a binder in an amount of 1 to 10 wt%, preferably 1 to 5 wt% per tablet. Further, it is also desirable to use calcium carboxymethyl cellulose, carmellose, less-substituted hydroxypropyl cellulose or insoluble polyvinyl pyrrolidone as a disintegrator in an amount of 5 to 20 wt%, preferably 5 to 10 wt% per tablet.

### treating agent (B);

Since the treating agent (B) of the present invention contains magnesium oxide particles and water-soluble dietary fibers, when it is orally administered before an examination or operation, even with a relatively small of a liquid (water), it can eject contents and water in the intestinal tract almost completely and can eject contents in the intestinal tract without greatly increasing the frequency of defecation, thereby making it possible to increase various examination precisions, and does not cause any troubles during an operation.

The treating agent (B) of the present invention contains magnesium oxide particles and water-soluble dietary fibers as the main components. It is desirable that the treating agent (B) of the present invention should be orally administered to ensure that the amount of the magnesium oxide particles becomes 1. 5 to 6 g, preferably 2 to 5 g and the amount of the water-soluble dietary fibers becomes 5 to 20 g, preferably 9 to 18 g per day for each person for each examination or each operation. At this point, the amount of water is about 600 to 1,500 ml, preferably 900 to 1,200 ml. The above-specified amounts of the magnesium oxide particles, the water-soluble dietary fibers and water are the total amounts used for each treatment (examination or operation) or the amounts per day and may be generally divided into two or three portions to be administered. For example, when they are divided into three portions to be administered per day, 0.5 to 2 g of the magnesium oxide and 1.6 to 6.6 g of the water-soluble dietary fibers should be administered together with 200 to 500 ml of water each time. When they are divided into portions to be administered, the portions of the treating agent and water to be administered each time do not need to be the same in quantity and may be different. The water-soluble dietary fibers may be administered 1 to 9 days before an examination or operation.

Since the treating agent (B) of the present invention contains the magnesium oxide particles, even when the amount of water to be orally taken is relatively small, the agent has excellent defecation and water ejection effects. The water-soluble dietary fibers do not interact with the magnesium oxide particles and greatly improves defection and intestinal tract cleaning effects due to a synergistic effect. Further, when the total amount of water for each treatment is about 1, 500 ml or less to administer these, the purpose can be completely accomplished.

The magnesium oxide particles contained in the treating agent (B) of the present invention exhibit antacid action in the stomach to neutralize gastric acid, and magnesium chloride produced by neutralization becomes a bicarbonate inside the intestine to exhibit laxative action. Therefore, as a treating agent having minimum antacid action produced by neutralization, the agent desirably contains magnesium oxide particles having an acid-neutralizing capacity for each treatment of 150 ml or more, preferably 700 to 3,000 ml as the consumption of 0.1 N hydrochloric acid.

The magnesium oxide particles used as the treating agent (B) have an average secondary particle diameter measured by a laser diffraction scattering method of 0.5 to 10 µm, preferably 1 to 7 µm.

The water-soluble dietary fibers contained in the treating agent (B) swell contents in the intestine and stimulate the intestinal tract to promote defecation and reduce the amount of remaining feces.

As the water-soluble dietary fibers contained in the treating agent (B), enzyme decomposed products of guar gum which have low viscosity, galactomannan and glucomannan, polydextrose, indigestible dextrin and reduced indigestible dextrin are preferred. Polydextrose, indigestible dextrin and reduced indigestible dextrin are particularly preferred.

The treating agent (B) of the present invention may contain the magnesium oxide particles and the water-soluble dietary fibers in various forms, desirably any one of a tablet, capsule, granule, powder, oral liquid, syrup and oral jelly. Out of these, the treating agent is preferably a tablet or granule from the viewpoints of ease of taking, formulation ease and preservability.

The water-soluble dietary fibers may be administered separately from the magnesium oxide particles.

For example, a preparation containing magnesium oxide particles and water-soluble dietary fibers is advantageous when it has (i) an average secondary particle diameter of the magnesium oxide particles contained therein of 0.5 to 10 µm, (ii) a content of the magnesium oxide particles contained therein of 3.0 to 60.0 wt%, preferably 7.0 to 54.5 wt% and (iii) a content (dry weight) of the water-soluble dietary fibers contained therein of 10.0 to 97.0 wt%, preferably 45.4 to 93.0 wt%.

### Examples and Reference Examples

### (i) Examination method

The large intestine is divided into 6 parts in order to evaluate remaining feces and remaining water in each part in five stages from an image of the large intestine at a supine position (SU) and a lateral recumbent position (PR) obtained from CT examination. Further, overall evaluation was carried out from the obtained evaluation results.

The large intestine is divided into the following six parts.
C: appendix
A: ascending colon
T: transverse colon
D: descending colon
S: sigmoid colon
R: rectum

The remaining feces were evaluated in five stages according to the existence of movement and a shape change.
0: difficult to evaluate
1: sticks to a mucous membrane
2: no movement and no shape change
3: moved and changed in shape
4: almost no remaining feces

The remaining water was evaluated in the following five stages according to its proportion to the circumference.
0: 4/4 of the circumference is under water (complete submersion).
1: 3/4 of the circumference is under water
2: 2/4 of the circumference is under water
3: 1/4 of the circumference is under water (submersion of semilunar pleat)
4: almost no submersion

The overall evaluation was made in the following five stages.
0: not useful
1: it cannot be said whether it is useful or not
2: rather useful
3: useful
4: very useful

### (ii) MgO tablet in use and its acid-neutralizing capacity

The MgO tablet in use is a preparation which contains MgO as an effective component and crystalline cellulose, croscarmellose and calcium stearate as additives. One MgO tablet in use contains 500 mg of MgO and has an acid-neutralizing capacity of 248 ml.

### (iii) Magnesium oxide and water-soluble dietary fiber-containing drink in use;

A magnesium oxide tablet which contains 500 mg of magnesium oxide was used as magnesium oxide for evaluation, and a drink prepared by dissolving 9 g of water-soluble dietary fibers in 500 ml was used as a water-soluble dietary fiber-containing drink. The magnesium oxide in use was magnesium oxide particles having an average secondary particle diameter specified in the present invention. The water-soluble dietary fibers in use were reduced indigestible dextrin described in the present invention.

### Examples 1 and 2

3 g of MgO for each examination was divided in two portions to be administered as the pretreatment of the examination. Three tablets (1.5 g of MgO) were orally administered together with 300 ml of water after dinner on the day before the examination, three tablets (1.5 g of MgO) were orally administered together with 300 ml of water before going to bed, and the CT examination of the large intestine was carried out on the examination day to obtain a cross-sectional image of the large intestine which was then processed by a computer to carry out the evaluation of remaining feces and remaining water as well as overall evaluation.

### Results

### Example 1

Remaining feces (SU): C (3) A (4) T (3) D (4) S (4) R (3)
Remaining feces (PR): C (3) A (4) T (3) D (4) S (4) R (3)
Remaining water (SU): C (4) A (4) T (4) D (4) S (4) R (4)
Remaining water (PR): C (4) A (4) T (4) D (4) S (4) R (4)
Overall evaluation: C (4) A (4) T (4) D (4) S (4) R (4)

### Example 2

Remaining feces (SU): C (1) A (4) T (4) D (4) S (4) R (4)
Remaining feces (PR): C (1) A (4) T (4) D (4) S (4) R (4)
Remaining water (SU): C (4) A (4) T (3) D (3) S (4) R (4)
Remaining water (PR): C (4) A (4) T (3) D (3) S (4) R (4)
Overall evaluation: C (4) A (4) T (4) D (4) S (4) R (4)

Although remaining feces or remaining water was observed, all the six parts of the large intestine were evaluated as (4) according to overall evaluation, and it was confirmed that the treating agent was very effective as a treating agent for the examination or operation of the large intestine.

### Reference example) Mg citrate preparation

### Reference Examples 1 and 2 (Mg citrate preparations)

A pharmaceutical product which contains Mg citrate as an effective component and is used to remove contents in the intestinal tract in the pretreatment of the examination of the large intestine was used as a Mg citrate preparation, and 68 g of Mg citrate was dissolved in water for each examination to prepare 1, 800 ml of a Mg citrate preparation to be administered as the pretreatment of the examination. 200 ml of the preparation was orally administered each time over about 1 hour from the day before the examination, and the CT examination of the large intestine was carried out on the examination day to obtain a cross-sectional image of the large intestine which was then processed by a computer to carry out the evaluation of remaining feces and remaining water as well as overall evaluation.

### Results

### Reference Example 1

Remaining feces (SU): C (4) A (4) T (4) D (3) S (4) R (4)
Remaining feces (PR): C (4) A (4) T (4) D (4) S (4) R (4)
Remaining water (SU): C (4) A (3) T (3) D (3) S (4) R (3)
Remaining water (PR): C (3) A (4) T (3) D (3) S (4) R (4)
Overall evaluation: C (4) A (4) T (4) D (4) S (4) R (4)

### Reference Example 2

Remaining feces (SU): C (3) A (4) T (2) D (4) S (4) R (4)
Remaining feces (PR): C (3) A (3) T (2) D (4) S (4) R (4)
Remaining water (SU): C (4) A (3) T (4) D (4) S (4) R (4)
Remaining water (PR): C (4) A (4) T (4) D (4) S (4) R (4)
Overall evaluation: C (4) A (4) T (2) D (4) S (4) R (4)

It was confirmed that magnesium oxide can remove contents in the large intestine like magnesium citrate and that it is extremely effective as a treating agent for the examination or operation of the large intestine as compared with the existing pharmaceutical products.

### Examples 3 and 4

As the pretreatment of the CT examination of the large intestine, 500 ml of a drink containing 9 g of water-soluble dietary fibers was administered on the day before the examination, 3 g of MgO for each examination was divided in two portions, that is, 3 tablets (1.5 g of MgO) were orally administered together with 300 ml of water after dinner on the day before the examination and 3 tablets (1.5 g of MgO) were orally administered together with 300 ml of water before going to bed, the defecation state was checked by inquiry before the examination on the examination day to evaluate the frequency of defecation, and then the CT examination of the large intestine was carried out to make overall evaluation. Results

### Example 3

77-year-old man, constipated, three times of defecation
Overall evaluation: C (4) A (4) T (4) D (4) S (4) R (4)

### Example 4

81-year-old man, not constipated, two times of defecation
Overall evaluation: C (4) A (4) T (4) D (4) S (4) R (4)

The CT examination of the large intestine could be carried out on both a subject complaining of constipation and a subject having no complaint without greatly increasing the frequency of defecation. According to overall evaluation, all the six parts of the large intestine were evaluated as (4), whereby it was confirmed that this treating agent was very effective as a treating agent for the examination or operation of the large intestine.

### Reference Examples 3 and 4 (magnesium citrate preparations)

A magnesium citrate preparation is a treating agent which has been used to remove contents in the intestinal tract for the examination or operation of the large intestine. About 1, 800 ml of the treating agent prepared by dissolving 68 g of magnesium citrate in water was administered for each examination as the pretreatment of the examination. 200 ml of the treating agent was orally administered over about 1 hour each time from the day before the examination, the defecation state was checked by inquiry on the examination day to evaluate the frequency of defecation, and then the CT examination of the large intestine was carried out to make overall evaluation.

### Results

### Reference Example 3

50-year-old woman, not constipated, 11 times of defecation
Overall evaluation: C (4) A (4) T (4) D (4) S (4) R (4) Reference Example 4
66-year-old woman, not constipated, 15 times of defecation

Overall evaluation: C (4) A (4) T (2) D (4) S (4) R (4) Although the treating agent containing magnesium oxide and water-soluble dietary fibers can remove contents in the large intestine like the magnesium citrate preparations, the frequency of defecation was apparently reduced as compared with existing pharmaceutical products, whereby it could be confirmed that a burden imposed on a subject by an increase in the frequency of defection is reduced surely.

### Examples 5 and 6

As the pretreatment of the CT examination of the large intestine, while 500 ml of a drink containing 9 g of water-soluble dietary fibers was administered on the day before the examination, 3 g of MgO for each examination was divided in two portions, that is, 3 tablets (1.5 g of MgO) were orally administered together with 300 ml of water after dinner on the day before the examination and 3 tablets (1.5 g of MgO) were orally administered together with 300 ml of water before going to bed, the defecation state was checked by inquiry before the examination on the examination day, and then the CT examination of the large intestine was carried out to make overall

### evaluation.

### Results

### Example 5

Remaining feces (SU): C (4) A (4) T (3) D (4) S (3) R (4)
Remaining feces (PR): C (3) A (4) T (4) D (4) S (4) R (4)
Remaining water (SU): C (4) A (3) T (3) D (0) S (3) R (0)
Remaining water (PR): C (4) A (4) T (3) D (4) S (3) R (4)
Overall evaluation: C (4) A (4) T (4) D (4) S (4) R (4)

### Example 6

Remaining feces (SU): C (4) A (4) T (4) D (4) S (4) R (4)
Remaining feces (PR): C (4) A (4) T (4) D (4) S (4) R (4)
Remaining water (SU): C (3) A (3) T (4) D (4) S (4) R (4)
Remaining water (PR): C (4) A (4) T (3) D (4) S (4) R (4)
Overall evaluation: C (4) A (4) T (4) D (4) S (4) R (4)

Although remaining feces or remaining water was partly observed, all the six parts of the large intestine were evaluated as (4) according to overall evaluation, whereby it was confirmed that the treating agent was very effective as a treating agent for the examination or operation of the large intestine.

### Examples 7 and 8

### (i) Examination method

Magnesium oxide particles and water-soluble dietary fibers were administered to rats for animal testing to check the defecation states after 9 hours. Laparotomy was made on the rats after 9 hours to measure the amount of remaining feces, the amount of dry feces and the content of water in the intestinal tract.

### (ii) Magnesium oxide particles and water-soluble dietary fibers in use

A preparation containing 1.4 g of magnesium oxide particles and 9 g of water-soluble dietary fiber powders was used to administer 700 mg/kg of the magnesium oxide particles and 4,500 mg/kg of the water-soluble dietary fiber powders to rats. The magnesium oxide in use was magnesium oxide particles having an average secondary particle diameter specified in the present invention. The water-soluble dietary fibers in use were reduced indigestible dextrin specified in the present invention.

### Examples 7 and 8

To clarify the effects on feces of the magnesium oxide particles and the water-soluble dietary fibers, as conditions that do not cause perfect excretion with a pharmacological dose of the magnesium oxide to the rat as an indicator, 700 mg/kg of magnesium oxide particles was administered, and powdery or liquid water-soluble dietary fibers or both of them were orally administered. The total suspension liquid doses to test groups were fixed. When the magnesium oxide particles and a mixture of the magnesium oxide particles and the water-soluble dietary fiber powders were administered, 5 ml/kg of them was administered, and when they were not administered, 0.05%CMC-NA was administered. When the water-soluble dietary fiber liquid was administered, 10 ml/kg of it was administered and when the water-soluble dietary fiber liquid was not administered, 10 ml/kg of water was administered. The defecation state was checked after 9 hours, and the amount of remaining feces, the amount of dry feces and the content of water in the large intestine of each rat were measured to evaluate the effects on the properties of feces of the magnesium oxide particles and the water-soluble dietary fibers.

### Results

### Example 7

The defecation states after 9 hours are shown below.

| Administered materials | | | |
|---|---|---|---|
| Magnesium oxide particles | Water-soluble dietary fiber powders | Water-soluble dietary fiber liquid | * |
| - | - | - | 0/5 |
| - | - | 4500 mg/kg | 0/5 |
| 700 mg/kg | - | - | 3/5 |
| 700 mg/kg | - | 4500 mg/kg | 2/5 |
| 700 mg/kg | 4500 mg/kg | - | 2/5 |
| 700 mg/kg | 4500 mg/kg | 4500 mg/kg | 4/5 |
| 700 mg/kg | 4500 mg/kg | 9000 mg/kg | 3/5 |

| | | | |
|---|---|---|---|
| *: Proportion having soft stool (number of rats having soft stool/number of test rats) | | | |

### Example 8

The amount of remaining feces, the amount of dry feces and the content of water in the large intestinal tract after 9 hours are shown below.

| Administered material | Amount of remaining feces (g) | Amount of dry feces | Content of water |
|---|---|---|---|
| none | 2.09 | 33% | 67% |
| Water-soluble dietary fiber liquid (4500 mg/kg) | 1.46 | 28% | 72% |
| Magnesium oxide (700 mg/kg) | 3.69 | 12% | 88% |
| Magnesium oxide (700 mg/kg) | 3.82 | 15% | 85% |
| Water-soluble dietary fiber liquid (4500 mg/kg) | | | |
| Magnesium oxide (700 mg/kg) | 2.81 | 15% | 85% |
| Water-soluble dietary fiber powders (4500 mg/kg) | | | |
| Magnesium oxide (700 mg/kg) | 2.29 | 14% | 86% |
| Water-soluble dietary fiber powders (4500 mg/kg) | | | |
| Water-soluble dietary fiber liquid (4500 mg/kg) | | | |
| Magnesium oxide (700 mg/kg) | 1.63 | 16% | 84% |
| Water-soluble dietary fiber powders (4500 mg/kg) | | | |
| Water-soluble dietary fiber liquid (9000 mg/kg) | | | |

It was confirmed that the amount of dry feces is reduced and the content of water is increased by administering the magnesium oxide particles and that the amount of remaining feces is reduced dependent on dose by administering the water-soluble dietary fibers.

That is, it was confirmed that a defecation effect is caused and the content of water in the feces is increased by administering the magnesium oxide particles, the amount of remaining feces can be reduced by administering the water-soluble dietary fibers, and a combination of the magnesium oxide particles and the water-soluble dietary fibers is suitable as a treating agent for the examination or operation of the large intestine.

It was also confirmed that the water-soluble dietary fibers to be combined may be solid or liquid.

### Example 9

### (i) Examination method

Magnesium oxide particles were administered to rats for animal testing to check the defecation states after 9 hours. At this point, the amount of water to be taken was increased and decreased to evaluate the effect on laxative action of the amount of water to be taken.

### (ii) Magnesium oxide particles in use

A preparation containing magnesium oxide as an effective component and crystalline cellulose, croscarmellose and calcium stearate as additives was used. The preparation in use was a tablet which contained 500 mg of magnesium oxide and had an acid-neutralizing capacity of 248 ml. The magnesium oxide in use is magnesium oxide particles having an average secondary particle diameter specified in the present invention.

### Example 9

To clarify the effects on feces of the magnesium oxide particles and the amount of ingested water, as conditions that do not cause perfect excretion with a pharmacological dose of the magnesium oxide to the rat as an indicator, 700 mg/kg of magnesium oxide particles was administered, and 1 ml/kg, 10 ml/kg and 35 ml/kg of water were orally administered. The defecation state of each test group was checked after 9 hours so as to calculate the water-like feces excretion rate and evaluate the effect on the properties of feces of the amount of ingested water when the magnesium oxide particles were administered.

### Results

### Example 9

The defecation states after 9 hours are shown below.

| Administered materials | | |
|---|---|---|
| Magnesium oxide | Water | Proportion causing diarrhea (number of rats suffering from diarrhea/number of test rats) |
| - | 1 mL/kg | 0/5 |
| - | 10 mL/kg | 0/5 |
| - | 35 mL/kg | 0/5 |
| 700 mg/kg | 1 mL/kg | 3/5 |
| 700 mg/kg | 10 mL/kg | 4/5 |
| 700 mg/kg | 35 mL/kg | 5/5 |

It was found that when defecation is to be promoted by magnesium oxide particles, a larger amount of water to be ingested at the time of administration is better and a smaller amount of water causes less defecation. When the preparation of the present invention is administered as a tablet, as the tablet disintegrates quickly, the amount of water to be taken can be reduced advantageously. However, when the amount of water to be taken is small, defecation is hardly caused, and it is important that the appropriate amount of water to be taken should be specified.

That is, it was confirmed that the specification of the amount of water to be taken for the examination or operation of the large intestine of a human is important as a treating agent.

## Claims

1. A treating agent (A) for the examination or operation of the large intestine, which contains 88 to 97 wt% of magnesium oxide particles and is used to be orally administered before an examination or operation.

2. The treating agent (A) according to claim 1, wherein the examination of the large intestine is CT examination, endoscopy or X-ray examination

3. The treating agent (A) according to claim 1, which contains 1.5 to 6 g of the magnesium oxide particles for each examination or each operation.

4. The treating agent (A) according to claim 1, which contains magnesium oxide particles having a one-time acid-neutralizing capacity of 150 ml or more as the consumption of 0.1 N hydrochloric acid.

5. The treating agent (A) according to claim 1, wherein the magnesium oxide particles have an average secondary particle diameter of 0.5 to 10 µm.

6. The treating agent (A) according to claim 1, which is a tablet, capsule, granule, powder, oral liquid, syrup or oral jelly which contains magnesium oxide particles.

7. The treating agent (A) according to claim 1, which is a tablet containing magnesium oxide particles as the main component and having (i) an average secondary particle diameter of the magnesium oxide particles contained therein of 0.5 to 10 µm, (ii) a content of the magnesium oxide particles contained therein of 88 to 97 wt% and (iii) a disintegration time of 10 seconds or less.

8. A treating agent (B) for the examination or operation of the large intestine, which contains magnesium oxide particles and water-soluble dietary fibers as effective components and is used to be orally administered before an examination or operation.

9. The treating agent (B) according to claim 8, wherein the examination of the large intestine is CT examination, endoscopy or X-ray examination.

10. The treating agent (B) according to claim 8, which contains 1.5 to 6 g of the magnesium oxide particles and 5 to 20 g of the water-soluble dietary fibers for each examination or each operation.

11. The treating agent (B) according to claim 8, which contains magnesium oxide having a one-time acid-neutralizing capacity of 150 ml or more as the consumption of 0.1 N hydrochloric acid.

12. The treating agent (B) according to claim 8, wherein the magnesium oxide particles have an average secondary particle diameter of 0.5 to 10 µm.

13. The treating agent (B) according to claim 8, wherein the water-soluble dietary fibers are at least one selected from the group consisting of reduced indigestible dextrin, indigestible dextrin and polydextrose.

14. The treating agent (B) according to claim 8, which is a tablet, capsule, granule, powder, oral liquid, syrup or oral jelly which contains magnesium oxide particles and water-soluble dietary fibers as effective components.

15. A treating method comprising the steps of:
orally administering the treating agent (A) or the treating agent (B); and
carrying out the examination or operation of the large intestine.

16. The treating method according to claim 15, wherein the examination of the large intestine is CT examination, endoscopy or X-ray examination.

17. The treating method according to claim 15, wherein the treating agent (A) or the treating agent (B) is orally administered to ensure that the amount of magnesium oxide particles becomes 1.5 to 6 g for each examination or each operation.

18. The treating method according to claim 15, wherein the treating agent (A) or the treating agent (B) is orally administered together with 600 to 1,500 ml of water for each examination or each operation.
